# EUROPEAN PATENT SPECIFICATION

(11) **EP 1 158 043 B1**
(45) Date of publication and mention of the grant of the patent: **30.12.2009**
(21) Application number: 00907974.0
(22) Date of filing: 09.03.2000
(51) Int. Cl.: C12N 1/21, C12N 15/00, C12P 13/08

(54) **PROCESS FOR PRODUCING L-LYSINE**
VERFAHREN ZUR HERSTELLUNG VON L-LYSIN
PROCEDE DE PRODUCTION DE L-LYSINE

(30) Priority: 09.03.1999 JP 6229299
(43) Date of publication of application: 28.11.2001
(73) Proprietor: Ajinomoto Co., Inc., Tokyo 104-0031 (JP)
(72) Inventor: ARAKI, Masayuki, Ajinomoto Co., Inc., Kawasaki-shi, Kanagawa 210-0801 (JP); SUGIMOTO, Masakazu, Ajinomoto Co., Inc., Kawasaki-shi, Kanagawa 210-0801 (JP); KIMURA, Eiichiro, Ajinomoto Co., Inc., Kawasaki-shi, Kanagawa 210-0801 (JP); NAKAMATSU, Tsuyoshi, Ajinomoto Co., Inc., Kawasaki-shi, Kanagawa 210-0801 (JP)
(74) Representative: Strehl Schübel-Hopf & Partner
(86) International application number: PCT/JP2000/001440
(87) International publication number: WO 2000/053726

(56) References cited:
- EP-A- 0 733 712
- EP-A- 0 756 007
- EP-A- 0 841 395
- EP-A- 1 029 919
- EP-A- 1 136 559
- WO-A-99/46363
- CA-A- 2 180 786
- DE-A- 199 007 347
- FI-A- 980 551
- JP-A- 61 268 185
- JP-A- 63 214 189
- KRAMER R: "Genetic and physiological approaches for the production of amino acids" JOURNAL OF BIOTECHNOLOGY, vol. 45, no. 1, 12 February 1996 (1996-02-12), pages 1-21, XP004036833 ISSN: 0168-1656
- CREMER J ET AL: "CONTROL OF THE LYSINE BIOSYNTHESIS SEQUENCE IN CORYNEBACTERIUM GLUTAMICUM AS ANALYZED BY OVEREXPRESSION OF THE INDIVIDUAL CORRESPONDING GENES" APPLIED AND ENVIRONMENTAL MICROBIOLOGY, vol. 57, no. 6, 1 June 1991 (1991-06-01), pages 1746-1752, XP000616281 ISSN: 0099-2240
- SCHRUMPF B ET AL: "A FUNCTIONALLY SPLIT PATHWAY FOR LYSINE SYNTHESIS IN CORYNEBACTERIUM GLUTAMICUM" JOURNAL OF BACTERIOLOGY, vol. 173, no. 14, July 1991 (1991-07), pages 4510-4516, XP000884180 ISSN: 0021-9193
- DATABASE BIOSIS 000093099296 BORMANN E. R. ET. AL.: 'Molecular analysis of the corynebacterium-glutamicum gdh gene encoding glumate dehydrogenase.' & MOLECULAR MICROBIOLOGY vol. 6, no. 3, 1992, pages 317 - 326

## Description

### Technical Field

The present invention relates to a method for producing L-lysine by fermentation. L-Lysine is widely used as a fodder additive.

### Background Art

L-Lysine have been industrially produced by fermentation by using coryneform bacteria belonging to the genus *Brevibacterium* or *Corynebacterium,* which have L-lysine productivity. As these coryneform bacteria, strains isolated from the nature world or artificial mutants of the strains have been used.

There have been disclosed various technologies to increase the L-lysine productivity by increasing activities of L-lysine biosynthesis enzymes by recombinant DNA technology. For example, it is known that in coryneform bacteria having L-lysine productivity, the L-lysine productivity of the bacteria is improved by introduction of a gene encoding aspartokinase desensitized in feedback inhibition by L-lysine and L-threonine (mutant *lysC*), a dihydrodipicolinate reductase gene (*dapB*), a dihydrodipicolinate synthase gene (*dapA*), a diaminopimelate decarboxylase gene (*lysA*), and a diaminopimelate dehydrogenase gene (*ddh*) (WO 96/40934); *lysA* and *ddh* (Japanese Patent Application Laid-Open No. 9-322774); *lysC*, *lysA* and a phosphoenolpyruvate carboxylase gene (*ppc*) (Japanese Patent Application Laid-Open No. 10-165180); or mutant *lysC*, *dapB*, *dapA, lysA* and an aspartate aminotransferase gene (*aspC*) (Japanese Patent Application Laid-Open No. 10-215883).

Also, it is known that in bacteria belonging to the genus *Escherichia*, L-lysine productivity is improved by enhancing *dapA*, mutant *lysC*, *dapB,* and a diaminopimelate dehydrogenase gene (*ddh*) (or a tetrahydrodipicolinate succinylase gene (*dapD*) and a succinyldiaminopimelate deacylase (*dapE*)) in order (WO 95/16042).

On the other hand, as technology for the purpose of improving glutamic acid productivity of glutamic acid-producing coryneform bacteria, cells possessing recombinant DNA containing a glutamate dehydrogenase gene originating from a bacterium belonging to the genus *Corynebacterium* are disclosed (Japanese Patent Application Laid-Open No. 61-268185). Glutamate dehydrogenase is an enzyme catalyzing a reaction of oxidation of α-ketoglutaric acid which is an intermediate of the tricarboxylic acid cycle to produce L-glutamic acid, and amplification of a gene encoding the enzyme improves L-glutamic acid productivity. Also, it has been shown that an amount of produced L-glutamic acid is increased by enhancing the glutamate dehydrogenase gene together with another glutamic acid biosynthesis enzyme gene (Japanese Patent Application Laid-Open No. 63-214189).

However, no relationship between glutamate dehydrogenase activity and L-lysine productivity has been known.

### Disclosure of Invention

An object of the present invention is to provide a method for producing L-lysine by fermentation, which is further improved compared with conventional ones.

The present inventors earnestly studied to solve the above problems. As a result, they assumed that supply of an amino group required for the reaction of producing aspartic acid from oxaloactic acid by aspartate aminotransferase may be a rate-limiting step of L-lysine production. The amino group is supplied through a deamination reaction from L-glutamic acid to α-ketoglutaric acid, which is accompanied with the above-mentioned reaction. Also, in the reaction of transferring an amino group to N-succinyl-2-amino-6-oxopimelic acid to produce L-lysine, L-glutamic acid is necessary as a second substrate. This reaction is catalyzed by succinyldiaminopimelate transaminase, and the amino group transferred in the reaction is also supplied by the deamination reaction from L-glutamic acid to α-ketoglutaric acid. Based on the assumption, taking notice of the reaction of glutamate dehydrogenase which makes the produced α-ketoglutaric acid back to glutamic acid, the present inventors have introduced a gene encoding glutamate dehydrogenase (hereinafter also referred to as "GDH") into a coryneform bacterium having L-lysine productivity to increase GDH activity, and found that L-lysine productivity of the bacterium can be improved. Based on this finding, the present invention has been completed.

Thus the present invention provides the coryneform bacterium and the method for producing L-lysine as defined in the claims.

### Brief Description of Drawings

Fig. 1 shows a process of construction of a plasmid pGDHm carrying *dgh*.
Fig. 2 shows a process of construction of a plasmid p299LYSA carrying *lysA.*
Fig. 3 shows a process of construction of a plasmid pCAB carrying mutant *lysC, dapA, dapB,* and Brevi.-ori.
Fig. 4 shows a process of construction of a plasmid pCABL carrying mutant *lysC, dapA, dapB, lysA,* and Brevi.-ori.

### Best Mode for Carrying out the Invention

### <1> The coryneform bacterium

The coryneform bacteirum is a coryneform bacterium which has L-lysine productivity, and in which intracellular GDH activity is increased.

The coryneform bacteria referred to in the present invention are a group of microorganisms as defined in Bergey's Manual of Determinative Bacteriology, 8th ed., p. 599 (1974), which are aerobic Gram-positive non-acid-fast rods having no spore-forming ability. The coryneform bacteria include bacteria belonging to the genus *Brevibacterium* having been hitherto classified into the genus *Brevibacterium* but united as bacteria belonging to the genus *Corynebacterium* at present (Int. J. Syst. Bacteriol., 41, 255 (1981)), and bacteria belonging to the genus *Brevibacterium* and the genus *Microbacterium* closely relative to bacteria belonging to the genus *Corynebacterium*. Examples of the coryneform bacterium suitably used to produce L-lysine include, for example, the following strains:
*Corynebacterium acetoacidophilum* ATCC 13870;
*Corynebacterium acetoglutamicum* ATCC 15806;
*Corynebacterium callunae* ATCC 15991;
*Corynebacterium glutamicum* ATCC 13032;
(*Brevibacterium divaricatum*) ATCC 14020;
(*Brevibacterium lactofermentum*) ATCC 13869;
(*Corynebacterium lilium*) ATCC 15990;
(*Brevibacterium flavum*) ATCC 14067;
*Corynebacterium melassecola* ATCC 17965;
*Brevibacterium saccharolyticum* ATCC 14066;
*Brevibacterium immariophilum* ATCC 14068;
*Brevibacterium roseum* ATCC 13825;
*Brevibacterium thiogenitalis* ATCC 19240;
*Microbacterium ammoniaphilum* ATCC 15354;
*Corynebacterium thermoaminogenes* AJ12340 (FERM BP-1539).

These can be furnished from, for example, the American Type Culture Collection. Each microorganism is assigned its registration number, and one can request provision of each microorganism by referring to its registration number. The registration numbers corresponding to the microorganisms are described in a catalog of the American Type Culture Collection. The AJ12340 strain has been deposited in National Institute of Bioscience and Human Technology, Agency of Industrial Science and Technology, Ministry of International Trade and Industry based on the Budapest Treaty.

Other than the bacterial strains described above, mutant strains having L-lysine productivity derived from these strains may be used for the present invention. Such artificial mutant strains include the followings: S-(2-aminoethyl)-cysteine (hereinafter abbreviated as "AEC") resistant mutant strains (For example, *Brevibacterium lactofermentum* AJ11082 (NRRL B-11470); see Japanese Patent Publication Nos. 56-1914, 56-1915, 57-14157, 57-14158, 57-30474, 58-10075, 59-4993, 61-35840, 62-24074, 62-36673, 5-11958, 7-112437, and 7-112438); mutant strains which require an amino acid such as L-homoserine for their growth (Japanese Patent Publication Nos. 48-28078 and 56-6499); mutant strains which exhibit resistance to AEC and require amino acids such as L-leucine, L-homoserine, L-proline, L-serine, L-arginine, L-alanine, and L-valine (United States Patent Nos. 3,708,395 and 3,825,472); L-lysine-producing mutant strains which exhibit resistance to DL-α-amino-ε-caprolactam, α-amino-lauryllactam, aspartate-analog, sulfa drug, quinoid, and N-lauroylleucine; L-lysine-producing mutant strains which exhibit resistance to inhibitors of oxyaloacetate decarboxylase or respiratory system enzymes (Japanese Patent Application Laid-open Nos. 50-53588, 50-31093, 52-102498, 53-9394, 53-86089, 55-9783, 55-9759, 56-32995 and 56-39778, and Japanese Patent Publication Nos. 53-43591 and 53-1833); L-lysine-producing mutant strains which require inositol or acetic acid (Japanese Patent Application Laid-open Nos. 55-9784 and 56-8692); L-lysine-producing mutant strains which exhibit sensitivity to fluoropyruvic acid or temperature not less than 34°C (Japanese Patent Application Laid-open Nos. 55-9783 and 53-86090); and mutant strains belonging to the genus *Brevibacterium* or *Corynebacterium* which exhibit resistance to ethylene glycol and produce L-lysine (United States Patent No. 4,411,997).

The terms "L-lysine productivity" used herein means an ability to accumulate a significant amount of L-lysine in a medium when the coryneform bacterium is cultured in the medium or to increase L-lysine content in cells.

### <2> Increase of GDH activity

In order to the GDH activity in a coryneform bacterial cell, a recombinant DNA can be prepared by ligating a fragment of a gene encoding GDH with a vector functioning in the bacterium, preferably a multi-copy vector, and introduced into a coryneform bacterium having L-lysine productivity to transform the bacterium. The copy number of the gene encoding GDH in the cell of the transformant is increased, and as a result, the GDH activity is increased.

As the gene encoding GDH, those originating from coryneform bacteria as well as those originating from other organism such as bacteria belonging to the genus *Escherichia* may be used.

The nucleotide sequence of a gene encoding GDH (*gdh* gene) originating from a coryneform bacterium has already been elucidated (Molecular Microbiology (1992) 6(3), 317-326). Therefore, the *gdh* gene can be obtained by PCR (polymerase chain reaction; see White, T.J. et al.; Trends Genet. 5, 185 (1985)) utilizing primers prepared based on the nucleotide sequence, for example, primers shown in SEQ ID NOS: 1 and 2 in Sequence Listing and coryneform bacterial chromosome DNA as a template. GDH-encoding genes originating from microorganisms other than coryneform bacteria may be obtained similarly.

The chromosome DNA can be prepared from a bacterium, which is a DNA donor, by the method of Saito and Miura (H. Saito and K. Kimura, Biochem. Biophys. Acta, 72, 619 (1963); Seibutsu Kogaku Jikkensho, The Society for Bioscience and Bioengineering, Japan ed., 97-98, Baifukan, 1992), for example.

It is preferred that the *gdh* gene amplified by PCR is ligated with a vector DNA autonomously replicable in a cell of *Escherichia coli* and/or coryneform bacteria to form a recombinant DNA, and the recombinant DNA is introduced into an *Escherichia coli* cell, whereby the subsequent procedures can be made easy. The vector autonomously replicable in *Escherichia coli* cells is preferably a plasmid vector, and preferably one autonomously replicable in a host cell. Examples thereof include pUC19, pUC18, pBR322, pHSG299, pHSG399, pHSG398, RSF1010 and so forth.

As a vector autonomously replicable in cells of coryneform bacteria, pAM330 (Japanese Patent Application Laid-Open No. 58-67699), pHM1519 (Japanese Patent Application Laid-Open No. 58-77895) and the like may be mentioned. Also, when a DNA fragment having ability to make a plasmid to be autonomously replicable in coryneform bacteria is taken out from these vectors and inserted into the above-mentioned vectors for Escherichia coli, they can be used as a shuttle vector autonomously replicable in both of *Escherichia coli* and coryneform bacteria.

Examples of the shuttle vector include the followings. The accession numbers of international depositary authorities, for microorganisms harboring the respective vectors are shown in parentheses. pAJ655: *Escherichia coli* AJ11882 (FERM BP-136)

*Corynebacterium glutamicum* SR8201 (ATCC 39135) pAJ1844: *Escherichia coli* AJ11883 (FERM BP-137)

*Corynebacterium glutamicum* SR8202 (ATCC 39136) pAJ611: *Escherichia coli* AJ11884 (FERM BP-138) pAJ3148: *Corynebacterium glutamicum* SR8203 (ATCC 39137) pAJ440: *Bacillus subtilis* AJ11901 (FERM BP-140) pHC4: *Escherichia coli* AJ12617 (FERM BP-3532)

These vectors are obtainable from the deposited microorganisms as follows. Cells collected at a logarithmic growth phase were lysed by using lysozyme and SDS, followed by separation from a lysate by centrifugation at 30,000 X g to obtain a supernatant. Polyethylene glycol is added to the supernatant, followed by fractionation and purification by means of cesium chloride-ethidium bromide equilibrium density gradient centrifugation.

In order to prepare recombinant DNA by ligating the GDH gene with a vector, the vector is digested by a restriction enzyme corresponding to the termini of a DNA fragment containing the GDH gene. Ligation is usually performed by using a ligase such as T4 DNA ligase.

To introduce the recombinant DNA prepared as described above into a coryneform bacterium, any known transformation methods can be employed. For example, a method of treating recipient cells with calcium chloride so as to increase the permeability of DNA, which has been reported for *Escherichia coli* K-12 (Mandel, M. and Higa, A., J. Mol. Biol., 53, 159 (1970)) and a method of preparing competent cells from cells which are at the growth phase followed by introducing the DNA thereinto, which has been reported for *Bacillus subtilis* (Duncan, C.H., Wilson, G.A. and Young, F.E., Gene, 1, 153 (1977)) may be used. Alternatively, a method of making DNA-recipient cells to be protoplasts or spheroplasts which can easily take up recombinant DNA followed by introducing the recombinant DNA thereinto (Chang, S. and Choen, S.N., Molec. Gen. Genet., 168, 111 (1979); Bibb,M.J., Ward, J.M. and Hopwood, O.A., Nature, 274, 398 (1978); Hinnen, A., Hicks, J.B. and Fink, G.R., Proc. Natl. Acad. Sci. USA, 75 1929 (1978)) may be also used. Also, the transformation of the coryneform bacteria may be performed according to the electric pulse method (Sugimoto et al., Japanese Patent Application Laid-Open No. 2-207791).

Increase of the GDH activity can also be achieved by allowing multiple copies of the GDH gene to be present on the chromosomal DNA of the host. In order to introduce multiple copies of the GDH gene into the chromosomal DNA of microorganisms belonging to the coryneform bacteria, the homologous recombination is carried out using a sequence whose multiple copies exist in the chromosomal DNA as targets. As the sequence whose multiple copies exist in the chromosomal DNA, repetitive DNA, inverted repeats existing at the end of a transposable element can be used. Alternatively, as disclosed in Japanese Patent Application Laid-Open No. 2-109985, it is possible to incorporate the *dgh* gene into transposon, and allow it to be transferred to introduce the multiple copies into the chromosomal DNA. By either method, the number of copies of the *dgh* gene in the transformant strain increases, and as a result, the GDH activity is increased.

In the coryneform bacteria of the present invention, in addition to the GDH activity, the activities of a gene encoding α-subunit or β-subunit of aspartokinase desenstized in synergistic feedback inhibition by L-lysine and L-threonine (International Publication No. WO 94/25605), a wild type phosphoenolpyruvate carboxylase gene originating from a coryneform bacterium (Japanese Patent Application Laid-Open No. 60-87788), a gent encoding wild type dihydrodipicolinate synthase originating from a coryneform bacterium (Japanese Patent Publication No. 6-55149), a dihydrodipicolinate reductase gene (Japanese Patent Application Laid-Open No. 7-75578) are also increased. Increase of these enzyme activity can be performed similarly to increase of the GDH activity, i.e by increasing the number of copies of the genes.

As the coryneform bacteria in which the activity of the enzyme of the L-lysine biosynthesis pathway is increased, there have been disclosed coryneform bacterium in which a gene encoding aspartokinase desensitized in feedback inhibition by L-lysine and L-threonine (mutant *lysC*), a dihydrodipicolinate reductase gene (*dapB*), a dihydrodipicolinate synthase gene (*dapA*), a diaminopimelate decarboxylase gene (*lysA*), and a diaminopimelate dehydrogenase gene (*ddh*) (WO 96/40934); *lysA* and *ddh* (Japanese Patent Application Laid-Open No. 9-322774); *lysC*, *lysA* and a phosphoenolpyruvate carboxylase gene (*ppc*) (Japanese Patent Application Laid-Open No. 10-165180); or mutant *lysC*, *dapB*, *dapA*, *lysA* and an aspartate aminotransferase gene (*aspC*) (Japanese Patent Application Laid-Open No. 10-215883) are introduced or amplified, respectively.

An AJ12691 strain obtained by introducing plasmid p399AK9B containing the mutant *lysC* into an AJ12036 strain (FERM BP-734) as a wild type strain of *Brevibacterium lactofermentum* has been deposited on April 10, 1992 under an accession number of FERM P-12918 in National Institute of Bioscience and Human Technology, Agency of Industrial Science and Technology, Ministry of International Trade and Industry (postal code: 305-8566, 1-3, Higashi 1-chome, Tsukuba-shi, Ibaraki-ken, Japan), transferred to international deposition based on the Budapest Treaty on February 10, 1995, and deposited under an accession number of FERM BP-4999.

A transformant strain AJ13107 obtained by introducing a plasmid pCRDAPB containing *dapB* into *E. coli* JM109 strain has been internationally deposited since May 26, 1995 under an accession number of FERM BP-5114 in National Institute of Bioscience and Human Technology, Agency of Industrial Science and Technology, Ministry of International Trade and Industry (postal code: 305-8566, 1-3, Higashi 1-chome, Tsukuba-shi, Ibaraki-ken, Japan) based on the Budapest Treaty.

A transformant strain AJ13106 obtained by introducing a plasmid pCRDAPA containing *dapA* into *E. coli* JM109 strain has been internationally deposited since May 26, 1995 under an accession number of FERM BP-5113 in National Institute of Bioscience and Human Technology, Agency of Industrial Science and Technology, Ministry of International Trade and Industry (postal code: 305-8566, 1-3, Higashi 1-chome, Tsukuba-shi, Ibaraki-ken, Japan) based on the Budapest Treaty.

Each gene of mutant *lysC*, *dapB* and *dapA* can be prepared from the deposited strains according to an ordinary method. Also, *lysA* can be obtained as a DNA fragment containing *argS* encoding arginyl-tRNA synthase, *lysA* and a promoter of an operon containing them, by PCR using oligonucleotides having nucleotide sequences shown in SEQ ID Nos: 3 and 4 in Sequence Listing as primers from chromosomal DNA of a coryneform bacterium, for example, a wild type strain of *Corynebacterium lactofermentum,* ATCC 13869 strain.

The aforementioned L-lysine biosynthesis pathway enzyme gene or genes and the *gdh* gene may be carried on an identical vector, or carried separately on two or more vectors.

In the coryneform bacteria, activity of an enzyme catalyzing reaction of producing a compound other than L-lysine and branching from the L-lysine biosynthesis pathway, may be decreased or deficient. As the enzyme catalyzing reaction of producing a compound other than L-lysine and branching from the L-lysine biosynthesis pathway, homoserine dehydrogenase may be mentioned (see WO 95/23864).

In this specification, the expression of "(enzyme) activity is increased" means that the intracellular enzymatic activity is higher that that of a wild strain, and that the intracellular enzymatic activity is higher than that of a strain before modification when a strain in which the intracellular activity is increased by modification by genetic recombinant technology or the like. Also, the expression of "(enzyme) activity is decreased" means that the intracellular enzymatic activity is lower that that of a wild strain, and that the intracellular enzymatic activity is lower than that of a strain before modification when a strain in which the intracellular activity is decreased by modification by genetic recombinantion technology or the like.

### <3> Method for producing L-lysine

By culturing the coryneform bacterium in which an intracellular activity of glutamate dehydrogenase is increased, and which has L-lysine productivity, in an appropriate medium, L-lysine is accumulated in the culture.

The medium to be used for L-lysine production by using the microorganism of the present invention may be an ordinary medium containing a carbon source, a nitrogen source, inorganic ions, and optionally other organic micronutrients.

As the carbon source, carbohydrates such as glucose, lactose, galactose, fructose, sucrose, molasses, and starch hydrolysate; alcohols such as ethanol and inositol, and organic acids such as acetic acid, fumaric acid, citric acid, and succinic acid may be used.

As the nitrogen source, inorganic ammonium salts such as ammonium sulfate, ammonium nitrate, ammonium chloride, ammonium phosphate and ammonium acetate; organic nitrogen such as ammonia, peptone, meat extract, yeast extract, yeast extract, corn steep liquor, and soybean hydrolysate; ammonia gas; and aqueous ammonia may be used.

As the inorganic ions, potassium phosphate, magnesium sulfate, iron ion, manganese ion and so on may be added in small amounts. As organic micronutrients, it is desirable to contain required substances such as vitamin B₁ or yeast extract or the like in appropriate amounts.

Cultivation is preferably carried out under an aerobic condition by shake culture, aeration agitation culture or the like for about 16 to 72 hours. The cultivation temperature is preferably controlled at 30°C to 45°C, and pH is preferably controlled at 5 to 9 during cultivation. Inorganic or organic, acidic or alkaline substances, or ammonia gas or the like can be used for pH adjustment.

L-Lysine can be collected from a culture by combining an ordinary ion exchange resin method, a precipitation method, and other known methods.

### EXAMPLES

The present invention will be more specifically explained below with reference to Examples.

### <1> Preparation of gdh gene originating from coryneform bacterium and construction of plasmid for introduction of gdh gene

Primers shown in SEQ ID Nos: 1 and 2 were prepared based on a known gdh gene sequence (Molecular Microbiology (1992) 6(3), 317-326) of *Corynebacterium glutamicum,* and PCR was performed by using chromosomal DNA of a wild type strain ATCC 13869 of *Brevibacterium lactofermentum* as a template to obtain a *gdh* gene fragment. DNA synthesis was performed accordance to an ordinary method with DNA synthesizer model 380B produced by Applied Biosystems, by using the phosphoamidite method (see Tetrahedron Letters (1981), 22, 1859). PCR reaction was performed with DNA Thermal Cycler Model PJ2000 produced by Takara Shuzo, by using Taq DNA polymerase in accordance with a method designated by the supplier. The amplified gene fragment was cloned into TA cloning vector pCR2.1 (produced by Invitrogen). The constructed plasmid was designated as pCRGDH.

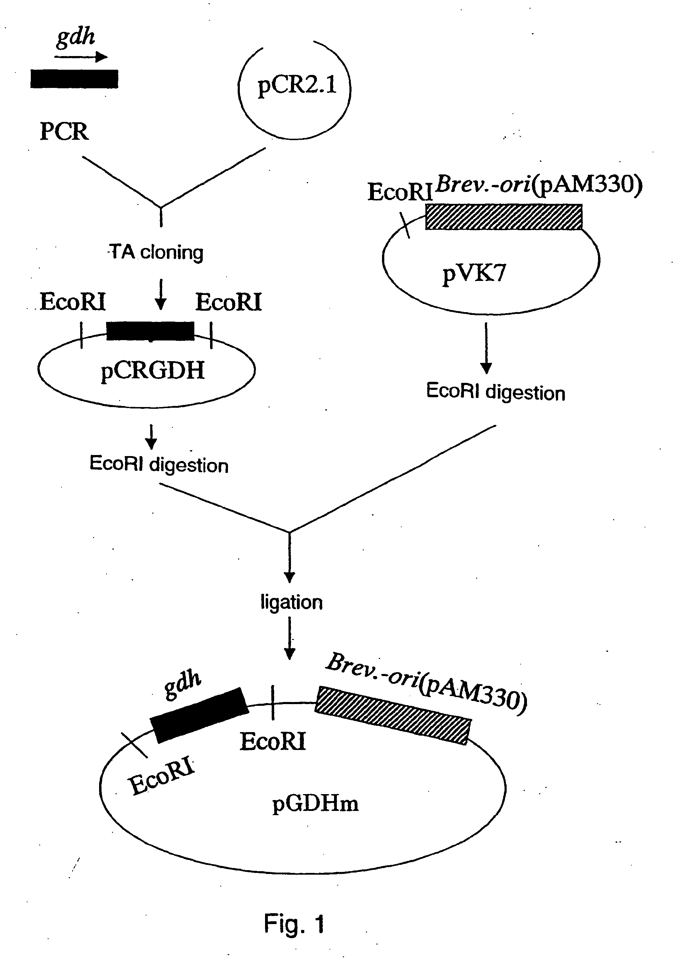

To introduce the cloned DNA fragment into a coryneform bacterium, the DNA fragment was ligated with a shuttle vector pVK7 (see Japanese Patent Application Laid-Open No. 10-215883). pCRGDH was digested with a restriction enzyme *Eco*RI (produced by Takara Shuzo), and it was ligated with pVK7 having been also digested with *Eco*RI. DNA was ligated by using DNA ligation kit (produced by Takara Shuzo) in accordance with a designated method. The constructed plasmid was designated as pGDHm. PGDHm has a kanamycin resistance gene as a marker. The process of construction of pGDHm is shown in Fig. 1.

pVK7 was constructed by ligating pHSG299, a vector for *E. coli* (Km^{r}; Takeshita, S. et al., Gene, 61, 63-74 (1987)) with pAM330, a cryptic plasmid for *Brevibacterium lactofermentum* as described below. pAM330 was prepared from *Brevibacterium lactofermentum* ATCC 13869 strain. pHSG299 was digested with a restriction enzyme resulting one cleavage site, AvaII (produced by Takara Shuzo), blunt-ended by using T4 DNA polymerase, and ligated with pAM330 having been digested with HindIII (produced by Takara Shuzo) and blunt-ended by using T4 DNA polymerase. Depending on orientation of the inserted pAM330 in pHSG299, the two obtained plasmids were designated as pVK6 and pVK7. pVK7 is autonomously replicable in both of *E. coli* and *Brevibacterium lactofermentum* and has a multiple cloning site originating from pHSG299 and *lacZ'*.

### <2> Introduction of gdh-carrying plasmid into L-lysine-producing Corynebacterium lactofermentum

### (1) Preparation of lysA and construction of plasmid containing lysA

Chromosomal DNA was prepared from a wild type strain ATCC 13869 of *Brevibacterium lactofermentum* in accordance with an ordinary method. A DNA fragment containing *argS*, *lysA*, and a promoter of an operon containing them was amplified from the chromosomal DNA in accordance with PCR. As for DNA primers used for amplification, synthetic DNA's of 23-mers having nucleotide sequences shown in SEQ ID NOs: 3 and 4 in Sequence Listing respectively were used in order to amplify a region of about 3.6 kb coding for arginyl-tRNA synthase and DDC on the basis of a sequence known for *Corynebacterium glutamicum* (see Molecular Microbiology, 4(11), 1819-1830 (1990); Molecular and General Genetics, 212, 112-119 (1988)). DNA was synthesized according to an ordinary method with DNA synthesizer model 380B produced by Applied Biosystems, by using the phosphoamidite method (see Tetrahedron Letters (1981), 22, 1859). The PCR reaction was performed with DNA Thermal Cycler Model PJ2000 produced by Takara Shuzo, by using Taq DNA polymerase in accordance with a method designated by the supplier. pHSG399 was used as a cloning vector for the amplified gene fragment of 3,579 bp. pHSG399 was digested with a restriction enzyme *Sma*I (produced by Takara Shuzo), and was ligated with the DNA fragment containing amplified *lysA.* A plasmid obtained as described above, which had *lysA* originating from ATCC 13869, was designated as p399LYSA.

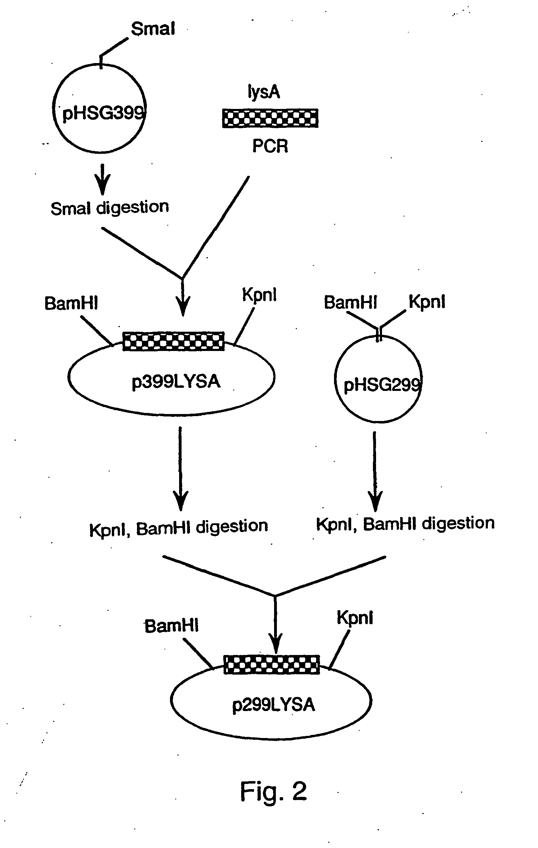

A DNA fragment containing lysA was extracted by digesting p399LYSA with *Kpn*I (produced by Takara Shuzo) and BamHI (produced by Takara Shuzo). This DNA fragment was ligated with pHSG299 having been digested with *Kpn*I and BamHI. An obtained plasmid was designated as p299LYSA. The process of construction of p299LYSA is shown in Fig. 2.

### (2) Construction of plasmid containing mutant lysC, dapA, and dapB together

The plasmid pCRDAPA containing *dapA* originating from *Brevibacterium lactofermentum,* disclosed in International Publication No. WO 96/40934, was digested with *Kpn*I and *Eco*RI to extract a DNA fragment containing *dapA* and the fragment was ligated with the vector plasmid pHSG399 having been digested with *Kpn*I and *Eco*RI. An obtained plasmid was designated as p399DPS. p399DPS was digested with *Eco*RI and *Sph*I followed by blunt end formation to extract a *dapA* gene fragment. This fragment was ligated with the p399AK9 containing mutant lysC originating from *Brevibacterium lactofermentum,* disclosed in International Publication No. WO 96/40934, having been digested with *Sal*I and blunt-ended to construct a plasmid p399CA in which mutant *lysC* and *dapA* co-existed.

pCRDAPA can be prepared from *E. coli* AJ13106 strain (FERM BP-5113) in accordance with an ordinary method. p399AK9 can be obtained by digesting p399AK9B with *Bam*HI to cut out a DNA fragment having an ability to make a plasmid autonomously replicable in coryneform bacteria (hereinafter referred to as "Brevi.-ori") and subjecting it to self-ligation. p399AK9B can be prepared from *E*. *coli* AJ12691 strain (FERM BP-4999) in accordance with an ordinary method.

The plasmid pCRDAPB carrying *dapB* originating from *Brevibacterium lactofermentum,* disclosed in International Publication No. WO 96/40934, was digested with *Eco*RI and blunt-ended, followed by digestion with *Sac*I to extract a DNA fragment of 2.0 kb containing *dapB.* The plasmid p399CA containing dap*A* and mutant *lysC* was digested with *Spe*I and blunt-ended, and was thereafter digested with *Sac*I and ligated with the extracted *dapB* fragment to obtain a plasmid containing mutant *lysC*, *dapA*, and *dapB.* This plasmid was designated as p399CAB. pCRDAPB can be prepared from *E*. *coli* AJ13107 strain (FERM BP-5114) in accordance with an ordinary method.

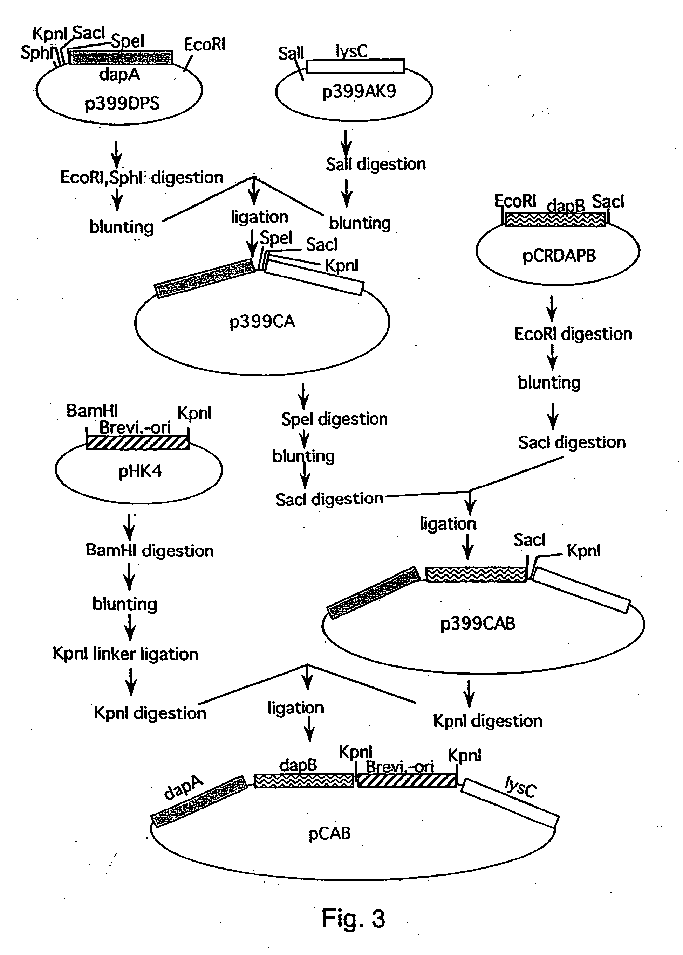

Next, Brevi.-ori was introduced into p399CAB. The plasmid pHK4 containing Brevi.-ori derived from pHM1519 was digested with a restriction enzyme *Bam*HI (produced by Takara Shuzo), and cleaved ends were blunted. Blunt end formation was performed by using DNA Blunting kit (produced by Takara Shuzo) in accordance with a designated method. After the blunt end formation, a phosphorylated *Kpn*I linker (produced by Takara Shuzo) was ligated to make modification so that the DNA fragment corresponding to the Brevi.-ori portion might be excised from pHK4 by digestion with only *Kpn*I. This plasmid was digested with *Kpn*I, and the generated Brevi.-ori DNA fragment was ligated with p399CAB having been also digested with *Kpn*I to construct a plasmid containing mutant *lysC*, *dapA,* and *dapB* together autonomously replicable in coryneform bacteria. The constructed plasmid was designated as pCAB. The process of construction of pCAB is shown in Fig. 3.

### (3) Construction of plasmid containing mutant lysC, dapA, dapB, and lysA together

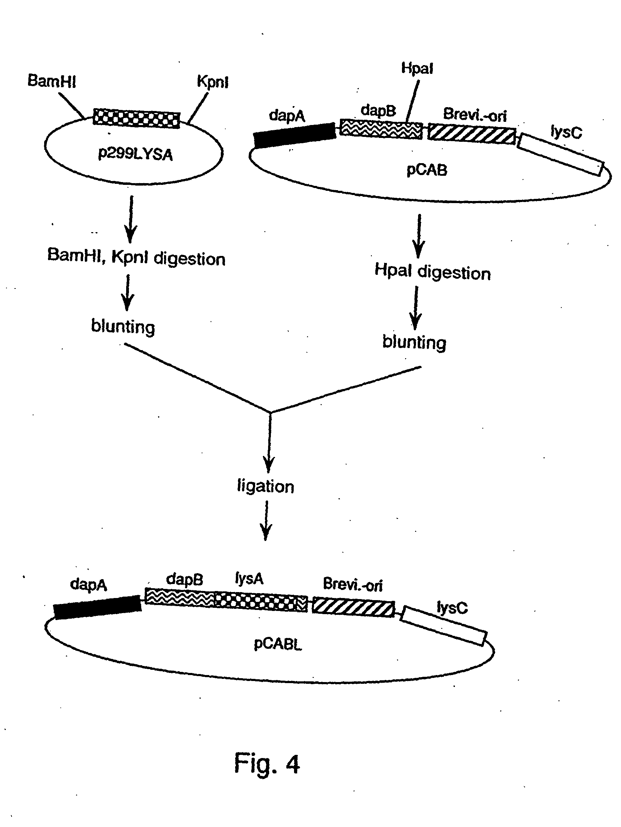

The plasmid p299LYSA containing *lysA* was digested with *Kpn*I and *Bam*HI and blunt-ended, and then a *lysA* gene fragment was extracted. This fragment was ligated with pCAB having been digested with *Hpa*I (produced by Takara Shuzo) and blunt-ended to construct a plasmid containing mutant *lysC*, *dapA*, *dapB,* and *lysA* together autonomously replicable in coryneform bacteria. The constructed plasmid was designated as pCABL. pCABL has a chloramphenicol resistance gene as a marker. The process of construction of pCABL is shown in Fig. 4. It is noted that the *lysA* gene fragment is inserted into a *Hpa*I site in a DNA fragment containing the *dapB* gene in pCABL, however, the *Hpa*I site is located upstream from a promoter for the *dapB* gene, and the *dapB* gene is not divided.

### (4) Preparation of L-lysine-producing coryneform bacterium transformed with gdh gene

An L-lysine-producing *Brevibacterium lactofermentum* AJ11082 (NRRL B-11470) was transformed with the plasmid pCABL prepared as described above to obtain AJ11082/pCABL. The AJ11802 strain has AEC resistance.

Then, the transformant AJ11082/pCABL was transformed with pGDHm. Because pCABL uses a replication origin derived from pHM1519 as a replication origin functional in cells of *Brevibacterium lactofermentum* and a chloramphenicol resistance gene as a marker gene, whereas pGDHm uses a replication origin derived from pAM330 as a replication origin functional in cells of *Brevibacterium lactofermentum* and a kanamycin resistance gene as a marker gene, the both of plasmids are stably harbored in cells of *Brevibacterium lactofermentum*. Thus, a strain AJ 11082/pCABL/pGDHm in which the plasmid containing L-lysine biosynthesis genes and the plasmid cotaining the *gdh* gene co-existed, was obtained.

### <3> Determination of GDH activity of each transformant

The GDH activities of AJ11082, AJ11082/pCABL and AJ11082/pCABL/pGDHm were determined. The determination of the GDH activity was performed in accordance with the method of E. R. Borman et al. (Molecular Microbiology (1992) 6(3), 317-326). About 10 ml of a culture solution cultured in a flask was centrifuged at 1500 rpm for 20 seconds to remove CaCO₃, and the supernatant was centrifuged at 3000 rpm for 6 minutes to collect cells. The collected cells were washed twice with 200 mM sodium phosphate buffer (pH 6.9) (KP buffer), and then suspended in 300 µl of KP buffer and subjected to ultrasonic disruption. The disruption solution was centrifuged at 3000 rpm for 10 minutes, and the supernatant was used as a crude enzyme solution for the following enzyme reaction experiments. The crude enzyme solution was added to a reaction solution (100 mM Tris-HCl (pH 8.0), 20 mM NH₄Cl, 10 mM α-ketogultaric acid, 0.25 mM NADPH) to measure change of absorbance at 340 nm. The results are shown in Table 1. In the table, *lysC** represents the mutant *lysC* gene. The GDH activity was shown as a relative value when the activity of AJ11082 was taken as 1. As a result, the GDH activity of AJ11082/pCABL/pGDHm was higher by about 7 times than that of AJ11082/pCABL.

**Table 1**

| Bacterial strain/plasmid | Introduced gene | GDH activity (relative value) |
|---|---|---|
| AJ11082 | | 1.0 |
| AJ11082/pCABL | *lysC*, dapA*, *dapB, lysA* | 1.0 |
| AJ11082/pCABL/pGDHm | *lysC*, dapA*, *dapB, lysA, gdh* | 7.3 |

### <4> Production of L-lysine

AJ11082, AJ11082/pCABL and AJ11082/pCABL/pGDHm were each cultivated in an L-lysine-producing medium to evaluate their L-lysine productivity. The L-lysine-producing medium had the following composition.

### [L-Lysine-producing medium]

The following components other than calcium carbonate (per liter) were dissolved and pH was adjusted to 8.0 with KOH. The medium was sterilized at 115°C for 15 minutes, and calcium carbonate (50 g) separately subjected to dry heat sterilization was added to the sterilized medium.

| | |
|---|---|
| Glucose | 100 g |
| (NH₄)₂SO₄ | 55 g |
| KH₂PO₄ | 1 g |
| MgSO₄·7H₂O | 1 g |
| Biotin | 500 µg |
| Thiamin | 2000 µg |
| FeSO₄·7H₂O | 0.01 g |
| MnSO₄·7H₂O | 0.01 g |
| Nicotinamide | 5 mg |
| Protein hydrolysate (Mamenou) | 30 ml |
| Calcium carbonate | 50 g |

Each of the transformants and the parent strain was inoculated to the medium having the composition described above to perform cultivation at 31.5°C with reciprocating shaking. The amount of produced L-lysine after 40 or 72 hours of cultivation, and the growth after 72 hours (OD₅₆₂) are shown in Table 2. The growth was quantitatively determined by measuring OD at 562 nm after 101-fold dilution. As a result, as shown in Table 2, improvement of the L-lysine productivity was observed in the GDH activity-increased strain.

**Table 2**

| Bacterial strain/plasmid | Introduced gene | Amount of produced L-lysine (g/L) After After | | Growth (OD₅₆₂ /101) |
|---|---|---|---|---|
| | | 40 hrs | 72 hrs | |
| AJ11082 | | 22.0 | 29.8 | 0.450 |
| AJ11082/pCABL | *lysC*, dapA, dapB, lysA* | 26.2 | 46.5 | 0.379 |
| AJ11082/pCABL/pGDHm | *lysC*, dapA, dapB, lysA, gdh* | 27.0 | 48.5 | 0.401 |

### Industrial Applicability

According to the present invention, the L-lysine productivity of a coryneform bacterium can be improved, thereby providing a more efficient method for producing L-lysine.

## Claims

1. A coryneform bacterium in which intracellular activities of glutamate dehydrogenase, the α-subunit or β-subunit of aspartokinase desensitized in synergistic feedback inhibition by L-lysine and L-threonine. dihydrodipicolinate reductase, dihydrodipicolinate synthase, and diaminopimelate decarboxylase are increased relative to an unmodified strain, and which has L-lysine productivity, wherein the activity of each enzyme is increased by increasing the number of copies of a gene encoding the enzyme in a cell of the bacterium.

2. The bacterium according to claim 1, wherein the gene encoding glutamate dehydrogenase originates from a coryneform bacterium.

3. A method for producing L-lysine comprising the steps of cultivating a coryneform bacterium according to claim 1 or 2 in a medium, to produce and accumulate L-lysine in a culture, and collecting L-lysine from the culture.

## Patentansprüche

1. Coryneformes Bakterium, worin die interzellulären Aktivitäten von Glutamatdehydrogenase, der gegenüber einer synergistischen Rückkopplungshemmung durch L-Lysin und L-Threonin unempfindlichen α-Untereinheit oder β-Untereinheit von Aspartokinase, Dihydrodipicolinatreductase, Dihydrodipicolinatsynthase und Diaminopimelatdecarboxylase im Vergleich zu einem nicht modifizierten Stamm erhöht sind und welches L-Lysin produziert, wobei die Aktivität jedes Enzyms durch Erhöhung der Anzahl der Kopien eines für das Enzym kodierenden Gens in einer Zelle des Bakteriums erhöht ist.

2. Bakterium nach Anspruch 1, wobei das für Glutamatdehydrogenase kodierende Gen aus einem coryneformen Bakterium stammt.

3. Verfahren zur Produktion von L-Lysin, welches die Stufen umfasst, bei denen ein coryneformes Bakterium nach Anspruch 1 oder 2 in einem Medium kultiviert wird, wobei L-Lysin in einer Kultur produziert und angehäuft wird, und L-Lysin aus der Kultur gewonnen wird.

## Revendications

1. Bactérie de type corynebacterium dans laquelle les activités intracellulairres de la glutamate déshydrogénase, de la sous-unité α ou de la sous-unité β de l'aspartokinase désensibilisée dans une inhibition rétroactive synergique par la L-lysine et la L-thréonine, de la dihydrodipicolinate réductase, de la dihydrodipicolinate synthase et de la diaminopimélate décarboxylase sont accrues par rapport à une souche non modifiée et qui a une productivité de L-lysine, où l'activité de chaque enzyme est accrue par accroissement du nombre de copies d'un gène codant l'enzyme dans une cellule de la bactérie.

2. Bactérie selon la revendication 1, où le gène codant la glutamate déshydrogénase provient d'une bactérie de type corynebacterium.

3. Procédé de production de L-lysine comprenant les étapes de culture d'une bactérie de type corynebacterium selon la revendication 1 ou 2 dans un milieu pour produire et accumuler de la L-lysine dans une culture et de recueil de la L-lysine dans la culture.


### Sequence Listing

<110> Ajinomoto Co., Inc.
<120> Method For Producing L-Lysine
<130> EPA-53734
<150> JP 11-62292
   <151> 1999-03-09
<160> 4
<170> PatentIn Ver. 2.0
<210> 1
   <211> 25
   <212> DNA
   <213> Artificial Sequence
<220>
   <223> Description of Artificial Sequence: primer for amplifying gdh gene
<400> 1
   gctagcctcg ggagctctct aggag 25
<210> 2
   <211> 25
   <212> DNA
   <213> Artificial Sequence
<220>
   <223> Description of Artificial Sequence: primer for amplifying gdh gene
<400> 2
   gatctttccc agactctggc cacgc 25
<210> 3
   <211> 23
   <212> DNA
   <213> Artificial Sequence
<220>
   <223> Description of Artificial Sequence: primer for amplifying lysA gen e
<400> 3
   gtggagccga ccattccgcg agg 23
<210> 4
   <211> 23
   <212> DNA
   <213> Artificial Sequence
<220>
   <223> Description of Artificial Sequence: primer for amplifying lysA gen e
<400> 4
   ccaaaaccgc cctccacggc gaa 23